# EUROPÄISCHE PATENTANMELDUNG

(11) **EP 1 623 716 A1**
(43) Veröffentlichungstag der Anmeldung: **08.02.2006**
(21) Anmeldenummer: 04020946.2
(22) Anmeldetag: 02.09.2004
(51) Int. Cl.: A61K 36/84, A61K 36/53, A61K 36/534, A61K 36/752, A61K 36/235, A61K 36/28, A61K 36/85, A61K 36/185, A61K 36/899, A61K 36/66, A61K 36/49, A61K 36/74, A61K 36/282, A61K 36/537, A61P 25/00

(54) **Beruhigende Pflanzenpräparate-Kombinationen mit aromatherapeutischen Komponenten, deren Herstellung und Verwendung**

(30) Priorität: 12.07.2004 DE 102004033785
(71) Anmelder: Finzelberg GmbH & Co. KG, 56626 Andernach (DE)
(72) Erfinder: Pischel, Ivo, Dr., 53547 Rossbach (DE); Feistel, Björn, Dr., 56626 Andernach (DE); Gaedcke, Frauke, Dr., 56323 Waldesch (DE)

(57) **Zusammenfassung**

Die vorliegende Erfindung betrifft ein Arzneimittel, ein Medizinprodukt, ein Nahrungsergänzungsmittel, eine diätetisches Lebensmittel, ergänzende bilanzierte Diäten und/oder ein Veterinärprodukt in Form beruhigender, anxiolytischer und schlafanstoßender Pflanzenpräparate-Kombinationen mit aromatherapeutischem Komponenten in dafür besonders geeigneten Darreichungsformen.
Solche Pflanzenpräparationen enthalten vorzugsweise wäßrige Extrakte aus Melisse, Hopfen und Hafer sowie Lavendelöl in einer oralen, insbesonderen lutsch- oder kaubaren Darreichungsform.

## Beschreibung

Die vorliegende Erfindung betrifft ein Arzneimittel, ein Medizinprodukt, ein Nahrungsergänzungsmittel, eine diätetisches Lebensmittel, ergänzende bilanzierte Diäten und/oder ein Veterinärprodukt in Form beruhigender, anxiolytischer und schlafanstoßender Pflanzenpräparate-Kombinationen mit aromatherapeutischen Komponenten in dafür besonders geeigneten Darreichungsformen.

Psychosomatische und psychische Erkrankungen, Syndrome und Leiden, die als Folge von oder im Zusammenhang mit körperlichen und vor allem mit geistiger Überbeanspruchung und Streß auftreten, zeigen besonders in den letzten Jahren ein stetiges Wachstum. Neben dem allgemein als Streß bezeichneten Zustand in allen Phasen des allgemeinen Anpassungssyndroms, sind hier des weiteren das Chronic Fatigue- sowie das Burnout-Syndrom, die Nieder- und Abgeschlagenheit, die Frühjahrsmüdigkeit, Angstzustände, Existenzängste und die sogenannte Manager-Krankheit zu nennen.
Auch die bereits im Kindesalter auftretenden Syndrome, wie Hyperaktivität und die Aufmerksamkeitsstörungen (ADD, Attention Deficit Disorder) zeigen eine wachsende Tendenz.

Zumeist werden zur Behandlung der obengenannten Erkrankungen, Syndrome und Leiden verschiedenste Sedativa und Psychopharmaka, wie Antidepressiva, Tranquilizer oder Psychosedativa, am häufigsten die tricyclische Verbindungen wie die Monoaminooxidaseinhibitoren (Fluoxitine, Prozac) oder die Benzodiazepinderivate, verordnet. Diese chemisch-synthetischen Verbindungen weisen jedoch auch eine Reihe von Nebenwirkungen, wie z.B. Mundtrockenheit, Miktionsstörungen, Verschlechterung der intellektuellen und motorischen Leistung auf. Aber auch offizinelle Arzneipflanzen haben in jeglicher Form von Extrakten, wie z. B. die von Baldrian, Passionblume, Hopfen und Rauschpfeffer, ihre pharmazeutischen Anwendungen.

Obwohl die chemisch-synthetischen zumeist stark wirksamen Arzneimittel mit relativ häufigen Nebenwirkungen und die pflanzenlichen Präparate mit in der Regel geringen Nebenwirkungen zur Behandlung der stressbedingten Erkrankungen gut geeignet sind, fehlen doch Pflanzenpräparate-Kombinationen auf Basis von Nahrungspflanzen, welche nicht das Nebenwirkungspotential dieser zumeist verordnungspflichtigen Arzneimittel besitzen und durch eine milde Wirkung eine ähnlich positive Beeinflußung der stressbedingten Syndrome erzielen können. Bei geeigneter Dosierung können diese Pflanzenpräparate-Kombinationen, aber auch stärkere Wirkungen entfalten, so daß sie insgesamt therapeutisch eine große Breite aufweisen. Die Erfindung hat zum Ziel, durch Kombination und Modifikation verschiedener Therapieansätze die letztgenannten Eigenschaften zu erreichen.

Diese Aufgabe wird erfindungsgemäß gelöst mit Kombinationen aus Pflanzenpräparationen, insbesondere Pflanzenextrakten, die in Darreichungsformen enthalten sind, die gleichzeitig ätherische Pflanzenöle enthalten.

Überraschenderweise zeigen diese Kombinationen aus Pflanzenpräparationen und ätherischen Ölen besondere synergistische Effekte bei der Anwendung als Arznei- oder Nahrungsergänzungsmittel oder bei den oben angeführten Erkrankungen, Syndromen und Leiden, die als Folge von oder im Zusammenhang mit körperlicher und vor allem mit geistiger Überbeanspruchung und Streß auftreten.

Die Pflanzenpräparationen enthalten entweder Pflanzenschnitte, -pulver und/oder Konzentrate und Pflanzenextrakte in jeglicher Form. Als Pflanzen werden z.B. Baldrian, Hopfen, Melisse, Hafer, Passionsblume, Rosmarin, Klatschmohn, Rotbusch, Linde, Meisterwurz und Schöllkraut verwendet.

Als aromatherapeutische Bestandteile sind ätherische Pflanzenöle von Lavendel, Melisse, Minze, Orangenblüten und Mandarinenöl sowie von Zitronenverbene, Majoran, Ylang Ylang, Fenchel, Estragon, Kamille, Eisenkraut, Lindenblüten, Indische Narde, Mandarine-Petitgrain und Muskatellersalbei geeignet.

Geeignete Darreichungsformen sind neben Teepräperationen z.B. Instantteepulver, Flüssigkeiten wie Tropfen oder Säfte, Hart- und Weichgelatinekapseln, Tabletten, Dragees und im Besonderen Lutschtabletten, Lutschpastillen, Bonbons und Wafer (wirkstoffhaltige Dünnfilmgele) auf Gelatine- oder Zellulosebasis.

Die lutschbaren und flüssigen Darreichungsformen haben gegenüber den verkapselten und festen Formen den Vorteil, daß die ätherische Pflanzenölkomponente, nach seiner Entfaltung als Aromatherapeutikum im Mund- und Nasenrachenraum zur Wirkung kommt.

Entgegen dem bisherigen Ansatz der Aromatherapie, der inhalativen Einnahme - dem inhalativen Kontakt mit ätherischen Ölkomponenten, wird durch die Darreichungsform einer z.B. lutschbaren Pastille ein besonders intensiver Kontakt zur Mundschleimhaut im Nasenrachenraum gewährleistet. Bei Verwendung solcher Lutschpastillen in einer klinischen Humanstudie wurde überraschenderweise schon bei einer sehr niedrigen Dosis eines erfindungsmäßen Nahrungsergänzungsmittels aus einer Kombinationen von Pflanzenextrakten und einem ätherischen Öl eine signifikante relaxierende und anxiolytische Wirkung gefunden, wie sie bisher nur durch oben beschriebene Arzneimittel (z.B. Valium) oder Phytopharmaka (z.B. Kava-Kava) erzielbar sind. Diese klinische Studie belegt durch EEG-Messungen (CATEEM-Untersuchung) die relaxierende und anxiolytische Wirkung eines Lutschpastillen-Präparates, erhältlich gemäß Beispiel 2, durch eine signifikante Erhöhung der α1, α2 und β1 - EEG-Frequenzbereiche gegenüber Plazebogabe (s. Abbildungen). Dieses sogenannte Brain-Mapping zeigt eine linkstemporale Betonung der Stimulierung, wobei α1 als Marker für eine relaxierende, α2 als Marker für das Working-Memory und β1 als Marker für die Anxiolyse angesehen werden. Ähnliche Aktivitäten werden auch durch Valium und Kava-Kava hervorgerufen.

Die nun folgenden Beispiele erläutern die Erfindung.

### Beispiel 1:

### Instanttee-Formulierung und deren Herstellung

Wässrige hergestellte Trockenextrakte aus Melissenblättern (215,8 kg), aus Hopfenblüten (35,9 kg) sowie von frischem Haferkraut (35,9 kg) werden nacheinander unter Rühren in Wasser gelöst (Trockensubstanzanteil ca. 15%). Anschließend erfolgt zu dieser Lösung die Zugabe die für die Trocknung und Aromatisierung benötigten technischen Hilfsstoffe (547,3 kg Maltodextrin, 7,7 kg Zuckercouleur gefärbt, 7,2 kg Gummi arabicum, 2,4 kg Aromamischung Vanille-Sahne). Die Mischung wird homogen gerührt bei einem Trockensubstanzanteil von ca. 40%. Diese Lösung wird zur entkeimenden Behandlung kurzzeit hocherhitzt.
Separat werden 2,0 kg Lavendelöl mit 0,1 kg Polysorbat 20 unter starkem Rühren miteinander homogenisiert. Diese Ölmischung wird unter ständigem Rühren zu der oben beschriebenen wässrigen Pflanzenextraktlösung mit Hilfsstoffen bei ca. 30-35°C gegeben. Diese Lösung wird im Sprühverfahren unter Schutzgasatmosphäre (CO₂) getrocknet. Die resultierende Trockenformulierung wird homogen gemischt und schutzgesiebt (1 mm). Diese als Instanttee verwendbare Trockenformulierung enthält in 1,2 g (Einzeldosierung pro Tasse) 195 mg der Trockenextraktemischung, bestehend aus 75% Melisseextrakt, 12,5% Hopfenextrakt und 12,5% Haferextrakt, sowie 2,4 mg mikroverkapseltes Lavendelöl.

### Beispiele 2:

### Pektinlutschpastillen

40 g handelsübliches Zitruspektin und 2 g Trinatriumcitrat werden mit 100 g Rohrzucker gut gemischt. Diese Mischung wird in 200 ml Wasser eingerührt und unter Rühren solange aufgekocht, bis sich das gesamte Pektin aufgelöst hat. Anschließend werden weitere 260 g Rohrzucker und 475 g Glukose-Fructose-Sirup zugegeben und bis auf ca. 80% Trockensubstanzgehalt eingeengt. Nun werden 45 g einer Mischung aus 50 % Melissenblätterextrakt, 25 % Hopfenzapfenextrakt und 25 % Haferfrischpflanzenextrakt und 1500 mg Lavendelöl sowie die Farb- und Aromastoffe zugefügt. Zur Einstellung des pH-Wertes auf 3,4-3,5 werden ca. 17 ml 50%ige Zitronensäurelösung zudosiert. Diese Masse wird bei ca. 95°C in geeignete Formen gegossen, so daß jeweils 2 g Pastillen erhalten werden. Insgesamt resultieren aus o.g. Ansatzmenge ca. 500 Pastillen von je zwei Gramm Nettogewicht enthaltend 90 mg Pflanzenextrakt-Mischung (Melisse/Hopfen/Hafer) sowie 3 mg Lavendelöl.

### Beispiel 3:

### Lutschpastillen auf Basis von Gummi Arabicum

In einer Rührapparatur werden bei 65°C unter Rühren 1100 kg Lösung hergestellt, die 33% Gummi Arabicum, 5% Sorbitol, 20 % Wasser und 42% Maltitol-Lösung enthält.

Dazu werden nacheinander eine parallel hergestellte Mischung aus 1,5 kg Lavendelöl und 0,5 kg Tween 20 sowie 15 kg einer Pflanzenextraktmischung, enthaltend 75 % Melissenblatt-, 12,5% Hopfen- und 12,5 % Haferextrakt, sowie nach Geschmack abgestimmte Mengen an Aromen und ggf. Süßstoffen eingerührt wird. Nach vollständiger Homogenisierung wird die Masse in Stärkeformen ausgegossen. Nach dem Trocknen bei 50°C wird das Endgewicht der Pastillen auf 2,0 g eingestellt, die 30 mg Trocknenextraktmischung und 3 mg Lavendelöl enthalten. Die Pastillen werden vom Stärkepulver getrennt und mit Trennmittel behandelt und abgefüllt. Es werden auf diese Weise ca. 5000 Pastillen erhalten.

### Beispiel 4:

### Kaugummi

100 g Chicle werden gepulvert, mit 300 g Zucker oder Zuckeraustauschstoff vermischt und in einer Abdampfschale solange erhitzt, bis die Masse weich wird. Sie wird dann unter Zugabe von 60 g Trockenextraktmischung aus Melisse, Hopfen und Hafer sowie 2 g Lavendelöl gut durchgearbeitet und auf eine mit Zucker bestreute Platte gebracht und bis zur Homogenität geknetet. Eine Aromatisierung kann im vorangegangenen Schritt ebenso erfolgen. Schließlich wird die Mischung in dünne Blätter ausgerollt und noch warm in flache Stangen geschnitten, indem man durch etwas Zuckerpulver das Ankleben der Masse an der Platte verhindert. Die so erhaltenen Kaugummi-Portionen sollen 2 bis 3 Gramm betragen.

### Beispiel 5:

### Einzelfall-Studie

Eine 55-jährige Frau (IK) lutschte nach einem arbeitsintensiven Tag eine Lutschpastille mit 30 mg Trockenextraktmischung sowie 1 mg Lavendelöl (hergestellt nach dem Herstellprinzip von Beispiel 3) und berichtete über eine entspannende und streßreduzierende Wirkung. Eine zweite und dritte Pastille steigerte die Wirkung ohne dabei sedierend zu wirken.

### Beispiel 6:

### Doppelblinde, plazebokontrollierte und randomisierte Humanstudie

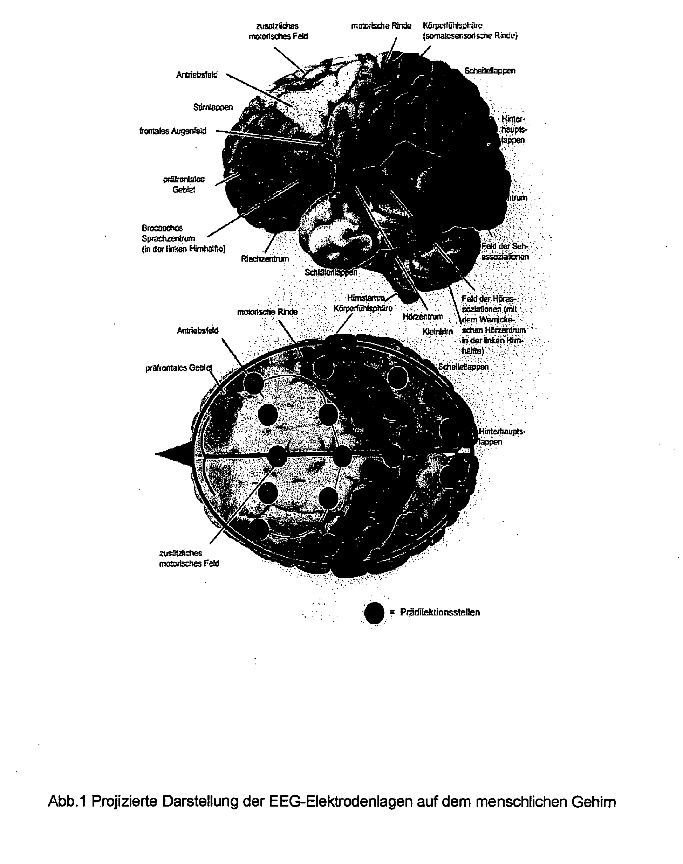

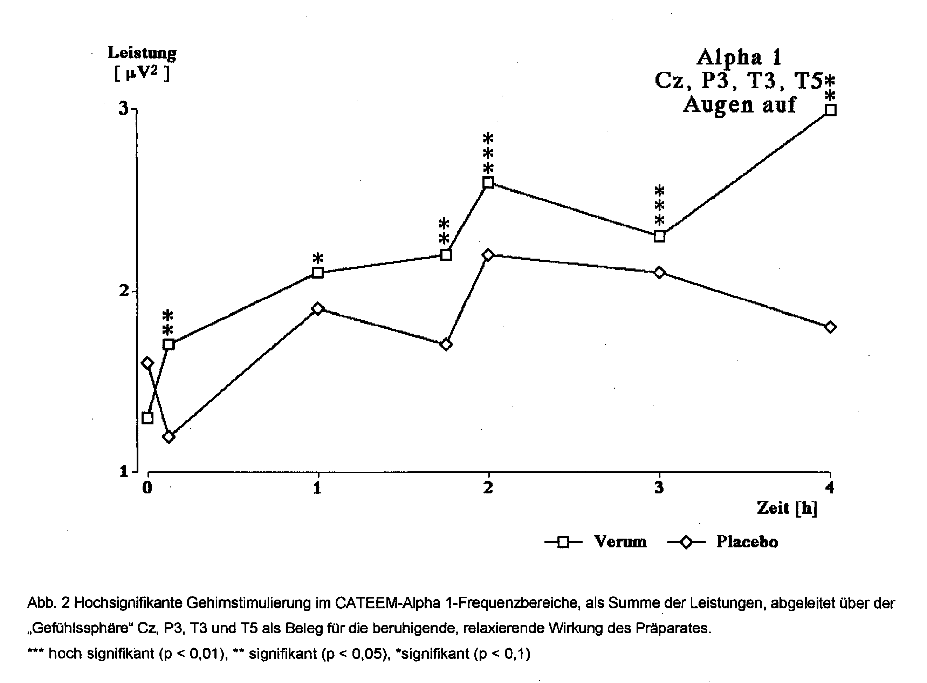

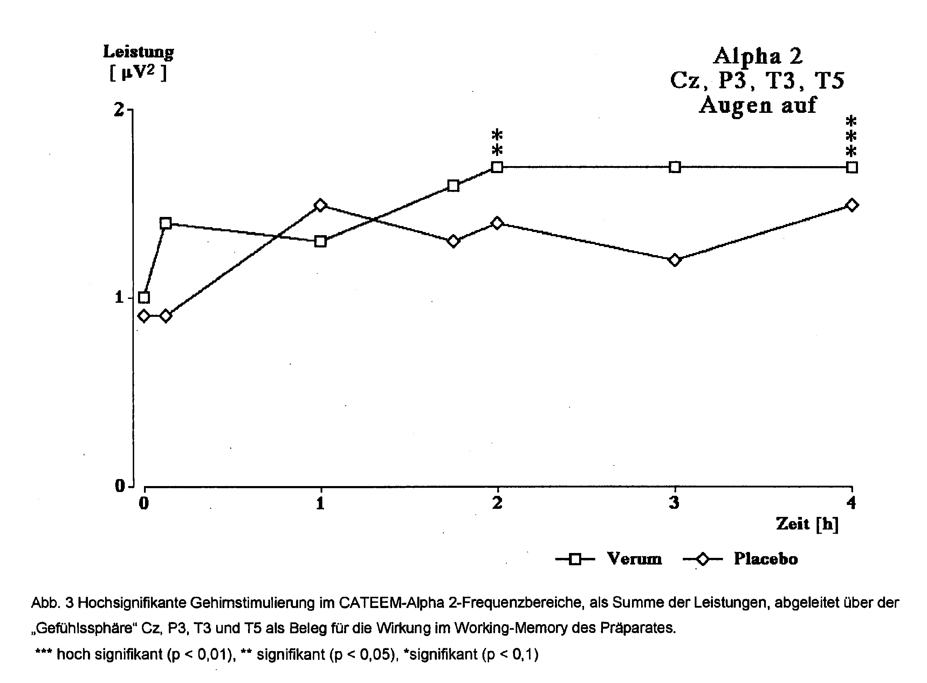

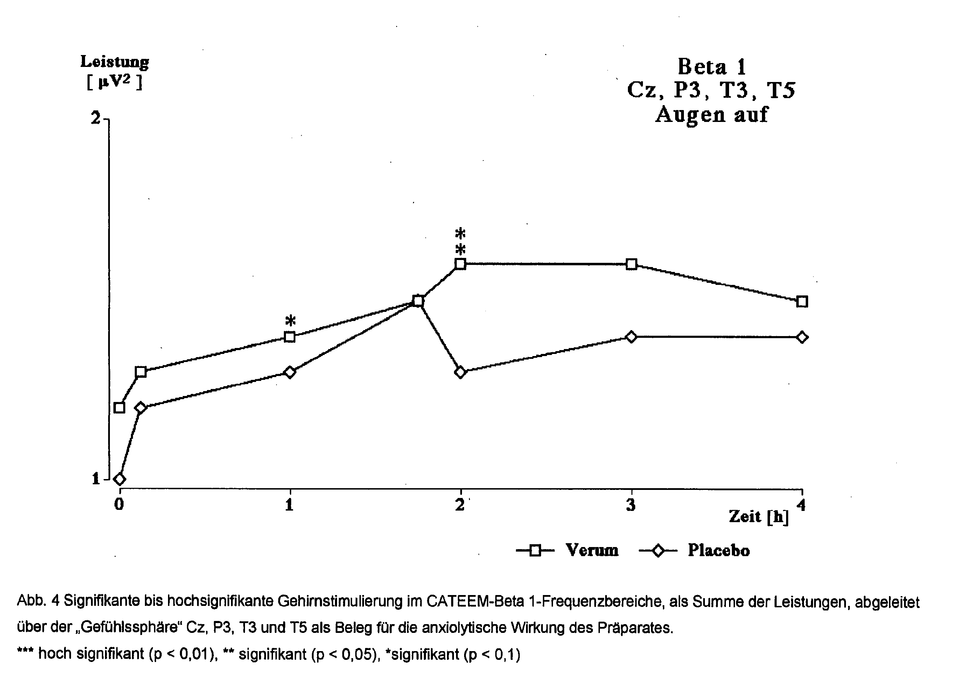

Herstellung einer erfindungsgemäßen Pflanzenpräparation nach Beispiel 2 als eine Lutschpastille mit 90 mg Trockenextraktkombination und 3 mg Lavendelöl. Testung dieser Darreichungsform mittels einer EEG-unterstützten klinischen Studie im Crossover-Design mit mindestens einer Woche Abstand. Es wurde für n=16 Probanden (m/w = 50/50) bei der Endauswertung eine signifikante Erhöhung der α1, α2 und β1 - EEG-Frequenzbereiche ermittelt. Der verwendeten EEG-Technik lag die CATEEM-Untersuchung zugrunde. Unter CATEEM versteht man eine Computer Aided Topographical Electro Encephalo Metry. Hierbei wird das normale EEG (d.h. die Hirnstromkurve) abgeleitet und dann mittels verschiedener mathematischer Verfahren (unter anderem der Fast-Fourier-Transformation) in eine Kartierung des Gehirns umgewandelt, die die verschiedenen Frequenzbänder unter den jeweiligen Elektroden abbildet. In der Neurologie wird auch von einem Brain Mapping durch EEG gesprochen.
Während der vierstündigen Studie lutschten die Probanden zu den Zeitpunkten Stunde 0 und 2 je eine Lutschpastille. Die EEG-Aufzeichnungen erfolgten zu mehreren Zeitpunkten.
Die Auswertung der Meßdaten erfolgte auf Basis der absoluten Werte zum jeweiligen Meßzeitpunkt (in MicroVolt²). Als Referenz dienten die Placebowerte. Die statistische Auswertung erfolgte mit dem nichtparametrischen Vorzeichentest. Insbesondere 2h nach der Pastilleneinnahme können signifikante Effekte der Hirnaktivitäten über den Elektroden Cz, P3, T3 und T5 (sogenannte Gefühlssphäre) gefunden werden. Diese stehen für einen relaxierenden Effekt. Die Ergebnisse dieser Studie sind in den Abbildungen 1 bis 4 dargestellt.

## Patentansprüche

1. Ein Arzneimittel, ein Medizinprodukt, ein Nahrungsergänzungsmittel, eine diätetisches Lebensmittel, ergänzende bilanzierte Diäten und/oder ein Veterinärprodukt in Form beruhigender, anxiolytischer und schlafanstoßender Pflanzenpräparate-Kombinationen mit aromatherapeutischem Komponenten.

2. Ein Arzneimittel, ein Medizinprodukt, ein Nahrungsergänzungsmittel, eine diätetisches Lebensmittel, ergänzende bilanzierte Diäten und/oder ein Veterinärprodukt nach Anspruch 1, **dadurch gekennzeichnet, daß** die Pflanzenpräparate Pflanzenschnitte, Teekonzentrate oder Pflanzenextrakte sind.

3. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 1 und 2 **dadurch gekennzeichnet, daß** es sich bei dem aromatherapeutische Komponenten um ätherische Pflanzenöle handelt.

4. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 1 bis 3, **dadurch gekennzeichnet, daß** Pflanzenpräparate von Pflanzen wie z.B. Baldrian, Hopfen, Melisse, Hafer, Passionsblume, Rosmarin, Klatschmohn, Rotbusch, Linde, Meisterwurz und Schöllkraut und die ätherische Pflanzenölkomponenten von Lavendel, Melisse, Minze, Orangenblüten und Mandarinenl sowie von Zitronenverbene, Majoran, Ylang Ylang, Fenchel, Estragon, Kamille, Eisenkraut, Lindenblüten, Indische Narde, Mandarine-Petitgrain und Muskatellersalbei stammen.

5. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 1 bis 4, **dadurch gekennzeichnet, daß** die Darreichungsformen neben Tropfen, Säfte, Tonika und Teepräparationen, Instantteepulver, Hartgelatinekapseln, Weichgelatinekapsel, Tabletten, Dragees, und im Besonderen Lutschtabletten, Lutschpastillen, Bonbons und Wafer (wirkstoffhaltige Dünnfilmgele) auf Gelatine- oder Zellulosebasis sind.

6. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 1 bis 5, **dadurch gekennzeichnet, daß** der Pflanzenpräparateanteil pro Einzeldosis 10 bis 500 mg, bevorzugt 10 bis 200mg an Pflanzenextrakten, und der ätherische Pflanzenöl von 0,1 - 25 mg, bevorzugt 0,5 - 5 mg reines ätherisches Öl beträgt. Die tägliche Dosis beträgt hierbei 1 bis 10-fache, bevorzugt 2 bis 4-fache der Einzeldosis.

7. Ein Arzneimittel, ein Medizinprodukt, ein Nahrungsergänzungsmittel, eine diätetisches Lebensmittel, ergänzende bilanzierte Diäten und/oder ein Veterinärprodukt nach dem Anspruchen 6, **dadurch gekennzeichnet, daß** es sich insbesondere um Kombinationen von wässrigen Pflanzenextrakten aus Melisse, Hopfen und Hafer sowie einer atherischen Ölkomponente , bevorzugt Lavendelöl, zusammensetzt.

8. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 6 und 7, **dadurch gekennzeichnet, daß** die Pflanzenpräparate-Kombination in einer oralen, insbesondere lutschbaren oder kaubaren Darreichungsform wie Lutschpastillen, Bonbons, Kaubonbons, Lutschtabletten, Strips oder Wafer eingebracht wird.

9. Ein Arzneimittel, ein Medizinprodukt, ein Nahrungsergänzungsmittel, eine diätetisches Lebensmittel, ergänzende bilanzierte Diäten und/oder ein Veterinärprodukt nach den Ansprüchen 6 bis 9, **dadurch gekennzeichnet, daß** eine Pflanzenpräparate-Kombination aus 10 - 100 mg an wässrigen Pflanzenextrakten aus Melisse, Hopfen und Hafer sowie 1 - 3 mg Lavendelöl in einer Lutschpastille als oraler Darreichungsform eingearbeitet werden.
